(19) Europäisches Patentamt
European Patent Office
Office européen des brevets

(11) EP 0 721 779 A2

(12) **EUROPÄISCHE PATENTANMELDUNG**

(43) Veröffentlichungstag:
**17.07.1996 Patentblatt 1996/29**

(51) Int Cl.[6]: **A61K 35/36**
// (A61K35/36, 31:20, 31:19)

(21) Anmeldenummer: **96100026.2**

(22) Anmeldetag: **03.01.1996**

(84) Benannte Vertragsstaaten:
**AT BE CH DE ES FR GB IT LI NL**

(30) Priorität: **12.01.1995 DE 19500702**

(71) Anmelder: **Beiersdorf Aktiengesellschaft**
**D-20245 Hamburg (DE)**

(72) Erfinder:
- **Klier, Manfred, Dr.**
  **D-21521 Aumühle (DE)**
- **Wolf, Florian Dr.**
  **D-20251 Hamburg (DE)**
- **Eitrich, Anja**
  **D-22587 Hamburg (DE)**

(54) **Wirkstoffkombination zur Verwendung gegen Superinfektionen, enthaltend Wollwachssäuregemische und alpha, omega-Alkandicarbonsäuren**

(57) Verwendung von Wirkstoffkombinationen aus

I) α,ω-Alkandicarbonsauren und

II) einem natürlichen Wollwachssäuregemisch oder einem durch Destillation eines natürlichen Wollwachssäuregemisches erhältlichen Wollwachssäuregemisch
als Wirkprinzip gegen Superinfektionen.

EP 0 721 779 A2

## Beschreibung

Wirkstoffkombinationen zur Verwendung gegen Superinfektionen, enthaltend Wollwachssäuregemische und α,ω-Alkandicarbonsäuren

Die gesunde menschliche Haut ist mit einer Vielzahl nichtpathogener Mikroorganismen besiedelt. Diese sogenannte Mikroflora der Haut ist nicht nur unschädlich, sie stellt einen wichtigen Schutz zur Abwehr opportunistischer oder pathogener Keime dar.

Bei bestehendem Primärinfekt, d.h., der normalen Keimbesiedelung der Haut, eintretende Neuinfektion mit hohen Keimzahlen eines oder mehrerer oft physiologischer Erreger, beispielsweise Staphylokokken, oft aber auch unphysiologischer Erreger, beispielsweise Candida albicans, kann bei Zusammentreffen ungünstiger Einflüssen eine "Superinfektion" der befallenen Haut auftreten. Die normale Mikroflora der Haut (oder eines anderen Körperorgans) wird dabei von dem Sekundärerreger regelrecht überwuchert.

Solche Superinfektionen können sich, in Abhängigkeit vom betreffenden Keim, in günstig verlaufenden Fällen in unangenehmen Hauterscheinungen (Juckreiz, unschönes äußeres Erscheinungsbild) äußern. In ungünstig verlaufenden Fällen können sie aber zu großflächiger Zerstörung der Haut führen, im schlimmsten Falle sogar im Tode des Patienten gipfeln.

Superinfektionen der vorab geschilderten Art sind z.B. beim Vollbild von AIDS häufig auftretende Sekundärerkrankungen.

An sich - jedenfalls in geringen Keimdichten -unschädliche, aber unter Umständen auch ausgesprochen pathogene Keime überwuchern auf diese Weise die gesunde Hautflora. Bei AIDS allerdings sind auch andere Körperorgane von Superinfektionen betroffen.

Ebenso werden derartige Superinfektionen bei einer Vielzahl dermatologischer Erkrankungen, z.B. atopischem Ekzem, Neurodermitis, Akne, seborrhoischer Dermatitis oder Psoriasis beobachtet. Auch viele medizinische und therapeutische Maßnahmen, z.B die Radio- oder Chemotherapie von Tumorerkrankungen, als Nebenwirkung hervorgerufene, medikamentös induzierte Immunsuppression oder aber systemische Antibiotikabehandlung, ebenso wie externe chemische oder physikalische Einflüsse (z.B. Umweltverschmutzung, Smog), fördern das Auftreten von Superinfektionen der äußeren und inneren Organe, insbesondere der Haut und der Schleimhäute.

Zwar ist es im Einzelfalle ohne weiteres möglich, Superinfektionen mit Antibiotika zu bekämpfen, meistens haben solche Substanzen aber den Nachteil unangenehmer Nebenwirkungen. Oft sind Patienten beispielsweise gegen Penicilline allergisch, weswegen eine entsprechende Behandlung sich in einem solchen Falle verbieten würde.

Ferner haben topisch verabreichte Antibiotika den Nachteil, daß sie die Hautflora nicht nur vom Sekundärerreger befreien, sondern auch die an sich physiologische Hautflora stark beeinträchtigen und der natürliche Heilungsprozeß auf diese Weise wieder gebremst wird.

Aufgabe der vorliegenden Erfindung war, die Nachteile des Standes der Technik zu beseitigen und Substanzen und Zubereitungen, solche Substanzen enthaltend, zur Verfügung zu stellen, durch deren Verwendung Superinfektionen geheilt werden können, wobei die physiologische Hautflora keine nenneswerte Einbußen erleidet.

Darüberhinaus war es Aufgabe der vorliegenden Erfindung, Substanzen und kosmetische bzw. dermatologische Zubereitungen, solche Substanzen enthaltend, zur Verfügung zu stellen, die prophylaktisch gegen Superinfektionen wirken.

Es wurde überraschend gefunden, und darin liegt die Lösung dieser Aufgabe, daß die Verwendung von Wirkstoffkombinationen aus

I) α,ω-Alkandicarbonsäuren und

II) einem natürlichen Wollwachssäuregemisch oder einem durch Destillation eines natürlichen Wollwachssäuregemisches erhältlichen Wollwachssäuregemisch

als Wirkprinzip gegen Superinfektionen, insbesondere gegen Superinfektionen der Haut den Nachteilen des Standes der Technik abhilft.

Die erfindungsgemäßen Wirkstoffgemische sind hervorragend wirksam gegen Superinfektionen, insbesondere gegen Staphylokokken, Pityrosporum ovale sowie Dermatophyten.

Zwar beschreibt die EP-OS 0 036 134 Desodorierende Zusammensetzungen, gekennzeichnet durch einen Gehalt an Derivaten mittel- bis längerkettiger Alkansäuren, welche auch die α,ω -Alkandicarbonsäuren der allgemeinen Formel

$$HOOC\text{- }(CH2)\ n\text{-}COOH$$

mit n = 4 bis 10 umfassen, ein Hinweis auf die hiermit vorgelegte Lehre findet sich in dieser Schrift jedoch nicht.

Ferner beschreibt die DE-OS 27 03 642 Mittel für die Körperhygiene, welche unter anderem gewisse α,ω-Alkandicarbonsäuren umfaßt, ein Hinweis auf die hiermit vorgelegte Lehre findet sich jedoch auch in dieser Schrift nicht.

Erfindungsgemäß werden die α,ω-Alkandicarbonsäuren bevorzugt gewählt aus der Gruppe der Substanzen, die von der generischen Formel

HOOC-(CH2)n-COOH beschrieben werden,

wobei n Zahlen von 1 bis 8 annehmen kann.

n = 1 : Malonsäure
n = 2 : Bernsteinsäure
n = 3 : Glutarsäure
n = 4 : Adipinsäure
n = 5 : Pimelinsäure
n = 6 : Korksäure
n = 7 : Azelainsäure
n = 8 : Sebacinsäure

Wollwachs oder Wollfett wird der bei der Rohwollwäsche anfallende fett- bis wachsartige Bestandteil der Rohschafwolle bezeichnet. Das Wollwachs besteht aus aus einem Gemisch von Fettsäureestern höherer Alkohole und aus freien Fettsäuren.

Die Hauptbestandteile der Wollwachssäuren sind

(a) gesättigte unsubstituierte Carbonsäuren, gemäß der Formel

CH3-(CH2)n-CH2-COOH,

(b) a-Hydroxycarbonsäuren, gemäß der Formel

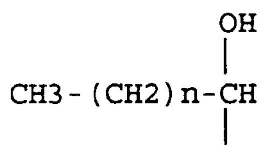

COOH,

(c) ω-Hydroxycarbonsäuren, gemäß der Formel

HO-CH2-(CH2)n-CH2-COOH,

(d) Isocarbonsäuren, gemäß der Formel

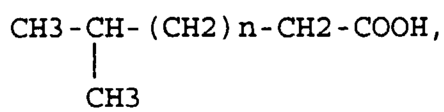

(e) α-Hydroxy-isocarbonsäuren, gemäß der Formel

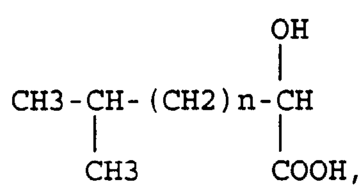

(f) ω-Hydroxy-isocarbonsäuren, gemäß der Formel

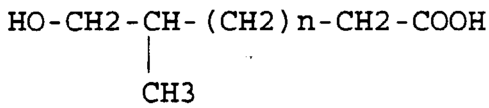

(g) Anteisocarbonsäuren, gemäß der Formel

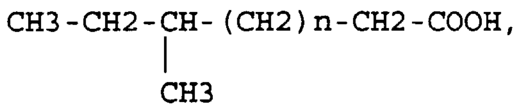

(h) α-Hydroxy-anteisocarbonsäuren, gemäß der Formel

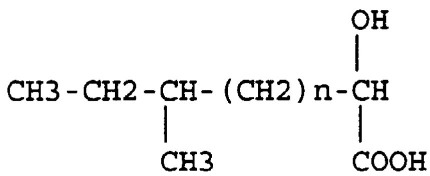

(i) ω-Hydroxy-anteisocarbonsäuren, gemäß der Formel

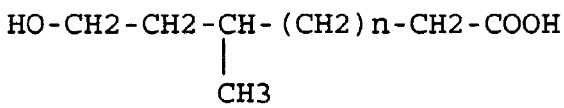

Dabei nimmt n gewöhnlich Werte von 7 - 31 an. Repräsentative Zusammensetzungen der Wollwachssäuren werden z.B. in "Parfümerie und Kosmetik", 59. Jahrgang, Nr.12/78, S.429, 430 sowie im "Lexikon der Hilfsstoffe für Pharmazie, Kosmetik und angrenzende Gebiete" von H.P.Fiedler, 1989, 3. Auflage, Editio Cantor Aulendorf, beschrieben.

Rohwollwachssäuren sind für kosmetische Zwecke nicht geeignet, statt ihrer werden für gewöhnlich destillierte Wollwachssäuren eingesetzt. Dieser Umstand und entsprechende Verfahren zur Raffinierung der Rohwollwachssäuren sind dem Fachmann bekannt.

Besonders vorteilhafte Verfahren zur Aufbereitung von Rohwollwachssäure sind beispielsweise der EP-OS 556 660 zu entnehmen.

Typischerweise bestehen Wollwachssäuren aus ca. 60 % gesättigten, unsubstituierten Carbonsäuren, ca. 30 % α-Hydroxycarbonsäuren und ca. 5 % ω-Hydroxycarbonsäuren, wobei der Rest von ca. 5 % im wesentlichen von den anderen vorgenannten Carbonsäuretypen gebildet wird.

Erfindungsgemäß besonders vorteilhaft ist eine Wollwachssäurefraktion, erhältlich durch Rohwollwachssäure durch Kurzwegdestillation im bei $10^{-1}$ bar aus dem Destillationstemperaturintervall von 150 - 200° C. Der Anteil an α-Hydroxycarbonsäuren beträgt dabei ca. 22 - 27 %. Solche Fraktionen zeichnen sich durch folgende kennzeichnende Parameter aus:

| | |
|---|---|
| Tropfpunkt : | 50 - 54° C |
| Säurezahl : | 166 - 170 |
| Verseifungszahl : | 175 - 190 |
| OH - Zahl : | 60 - 80 |
| Jodzahl : | 7 - 12 |

Zwar ist aus dem Aufsatz "Antimicrobial Factors in Wool Wax" (Australian Journal of Chemistry, 1971, 24, Seiten 153 ff.) bekannt, daß in manchen Wollwachschargen antimikrobielle Faktoren enthalten sind. Ein Hinweis in Richtung der vorliegenden Erfindung findet sich am angegebenen Orte jedoch nicht.

Als besonders vorteilhafte Verkörperung der vorliegenden Erfindung werden Wirkstoffkombinationen mit einem Gehalt an

I) Adipinsäure und

II) einem natürlichen oder durch Destillation aufbereiteten Gemisch aus Wollwachssäuren

angesehen.

Es ist von Vorteil, den Gehalt an

I) α,ω-Alkandicarbonsäuren und

II) Einem natürlichen oder durch Destillation aufbereiteten Gemisch aus Wollwachssäuren

so zu wählen, daß Verhältnisse von I) und II) wie 5 : 1 bis 1 : 5, insbesondere wie etwa 1 : 1 entstehen.

Die erfindungsgemäßen Zubereitungen sind besonders vorteilhaft dadurch gekennzeichnet, daß das natürliche Wollwachssäuregemisch oder das durch Destillation eines natürlichen Wollwachssäuregemisches erhältliche Wollwachssäuregemisch in Konzentrationen von 0,05 - 10,00 Gew.-%, bevorzugt 0,1 - 5,0 Gew.-%, vorliegt, jeweils bezogen auf das Gesamtgewicht der Zubereitungen.

Die erfindungsgemäßen Zubereitungen sind besonders vorteilhaft dadurch gekennzeichnet, daß die α,ω-Alkandicarbonsäure oder die α,ω-Alkandicarbonsäuren in Konzentrationen von 0,01 - 10,00 Gew.-%, bevorzugt 0,05 - 5,0 Gew.-%, vorliegen, jeweils bezogen auf das Gesamtgewicht der Zubereitungen.

Erfindungsgemäße dermatologische Zubereitungen können in Form von Aerosolen, also aus Aerosolbehältern, Quetschflaschen oder durch eine Pumpvorrichtung versprühbaren Präparaten vorliegen oder in Form von mittels Roll-on-Vorrichtungen auftragbaren flüssigen Zusammensetzungen, jedoch auch in Form von aus normalen Flaschen und Behältern auftragbaren W/O- oder O/W-Emulsionen, z.B. Cremes oder Lotionen. Weiterhin können die Zubereitungen vorteilhaft in Form von Tinkturen, Shampoos, Wasch-, Dusch- oder Badezubereitungen oder Pudern vorliegen.

Als übliche kosmetische Trägerstoffe zur Herstellung der erfindungsgemäßen dermatologischen Zubereitungen können neben Wasser, Ethanol und Isopropanol, Glycerin und Propylenglykol hautpflegende Fett- oder fettähnliche Stoffe, wie Ölsäuredecylester, Cetylalkohol, Cetylstearylalkohol und 2-Octyldodecanol, in den für solche Präparate üblichen Mengenverhältnissen eingesetzt werden sowie schleimbildende Stoffe und Verdickungsmittel, z.B. Hydroxyethyl- oder Hydroxypropylcellulose, Polyacrylsäure, Polyvinylpyrrolidon, daneben aber auch in kleinen Mengen cyclische Silikonöle (Polydimethylsiloxane) sowie flüssige Polymethylphenylsiloxane niedriger Viskosiät.

Als Treibmittel für erfindungsgemäße, aus Aerosolbehältern versprühbare dermatologische Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorchlorkohlenwasserstoffe (FCKW).

Als Emulgatoren zur Herstellung der erfindungsgemäßen dermatologischen Zubereitungen, haben sich nichtionogene Typen, wie Polyoxyethylen-Fettalkoholether, z.B. Cetylstearylalkoholpolyethylenglykolether mit 12 bzw. 20 angelagerten Ethylenoxid-Einheiten pro Molekül, Cetostearylalkohol sowie Sorbitanester und Sorbitanester-Ethylenoxid-Verbindungen (z.B. Sorbitanmonostearat und Polyoxyethylensorbitanmonostearat) und langkettige höhermolekulare wachsartige Polyglykolether als geeignet erwiesen.

Zusätzlich zu den genannten Bestandteilen können den erfindungsgemäßen dermatologischen Zubereitungen, deren pH-Wert vorzugsweise z.B. durch übliche Puffergemische auf 4,0 bis 7,5 insbesondere 5,0 bis 6,5, eingestellt wird, Parfüm, Farbstoffe, Antioxidantien, Suspendiermittel, Puffergemische oder andere übliche kosmetische oder dermatologische Grundstoffe beigemischt werden.

Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall)-Chelatoren (z.B,α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure,

Ölsäure), Folsäure und deren Derivate, Ubichinon und Ubichinol und deren Derivate, Vitamin C und Derivate (z.B. Ascorbylpalmitat, Mg - Ascorbylphosphat, Ascorbylacetat), Tocopherole und Derivate (z.B. Vitamin E - acetat), Vitamin A und Derivate (Vitamin A - palmitat) sowie Konyferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO4) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zukker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Es ist für die vorliegende Erfindung vorteilhaft, daß der pH-Wert der erfindungsgemäßen dermatologischen Zubereitungen kleiner als 8 ist. pH-Werte, die leicht höher sind als 7, aber kleiner als 7,5 können dabei im allgemeinen toleriert werden. Jedenfalls ist für ein gegebenes Fettsäuregemisch im Einzelfalle durch einfaches Ausprobieren, ohne erfinderisches Dazutun, leicht zu ermitteln, welche exakte obere pH-Grenze zu beachten ist.

Vorteilhaft wird der pH-Wert der erfindungsgemäßen Formulierungen im sauren bis sehr schwach alkalischen Bereich kleiner als 8 eingestellt, bevorzugt von 4,0 - 7,5 , besonders bevorzugt von 5,0 - 6,5.

Die jeweils einzusetzenden Mengen an Hilfs- Zusatz- und Trägerstoffen und gegebenenfalls Parfüm können in Abhängigkeit von der Art des jeweiligen Produktes vom Fachmann durch einfaches Ausprobieren leicht ermittelt werden.

Die Herstellung der erfindungsgemäßen dermatologischen Zubereitungen erfolgt, abgesehen von speziellen Zubereitungen, die in den Beispielen jeweils gesondert vermerkt sind, in üblicher Weise, zumeist durch einfaches Vermischen unter Rühren, gegebenenfalls unter leichter Erwärmung. Sie bietet keine Schwierigkeiten. Für Emulsionen werden Fettphase und die Wasserphase z.B. separat, gegebenenfalls unter Erwärmen hergestellt und dann emulgiert.

Ansonsten sind die üblichen Maßregeln für das Zusammenstellen von galenischen Formulierungen zu beachten, die dem Fachmann geläufig sind.

Sollen die erfindungsgemäßen Kombinationen in Puder eingearbeitet werden, so können die Suspensionsgrundlagen dafür vorteilhaft gewählt werden aus der Gruppe

Kieselsäuregele (z.B. solche die unter dem Handelsnamen Aerosil® erhältlich sind), Kieselgur, Talkum, modifizierte Stärke, Titandioxid, Seidenpulver, Nylonpulver, Polyethylenpulver und verwandten Stoffen.

Es folgen vorteilhafte Ausführungsbeispiele der vorliegenden Erfindung. Die Mengenangaben beziehen sich stets auf Gewichts-%, wenn nichts Anderes angegeben wird. In den Beispielen bedeutet der Begriff "WWS" eine Wollwachssäurefraktion, welche gewonnen wurde aus Rohwollwachssäure durch Kurzwegdestillation im bei $10^{-1}$ bar aus dem Destillationstemperaturintervall von 150 - 200° C. Der Anteil an $\alpha$ -Hydroxycarbonsäuren beträgt dabei ca. 22 - 27 %

<u>Beispiel 1 - 3</u>

| Pumpspray | 1 | 2 | 3 |
|---|---|---|---|
| Ethanol | 60,00 | 60,00 | 60,00 |
| Propylenglycol | 3,00 | 3,00 | 3,00 |
| PEG-40-hydriertes Rizinusöl | 2,50 | 2,50 | 2,00 |
| Adipinsäure | 0,45 | - | - |
| Azelainsäure | - | 0,35 | 0,30 |
| WWS | 0,30 | 0,35 | 0,35 |
| Parfum | q.s. | q.s. | q.s. |
| Wasser, NaOH 10-%ig ad pH 5 | ------------ad 100,00------------ | | |

Beispiel 4 - 5

| Roll-on Gel | 4 | 5 |
|---|---|---|
| Ethanol | 50,00 | 45,00 |
| Hydroxyethylcellulose | 0,50 | 0,50 |
| Steareth-20 ("Brij 78®") | 1,50 | 1,50 |
| Adipinsäure | - | 0,50 |
| Azelainsäure | 0,45 | - |
| WWS | 0,35 | 0,30 |
| Parfum | q.s. | q.s. |
| Wasser, NaOH 10-%ig ad pH 5 | -----ad 100,00------ | |

Beispiel 6 - 7

| Roll-On Emulsion (O/W) | 6 | 7 |
|---|---|---|
| Steareth-10 ("Brij 76®") | 4,00 | 4,00 |
| Cetylalkohol | 2,00 | 1,50 |
| Mineralöl DAB 9 | 7,00 | 7,00 |
| PPG-15 Stearylether | 4,50 | 4,50 |
| Methylparaben | 0,20 | 0,20 |
| Dipropylenglycol | 2,50 | 2,50 |
| Adipinsäure | 0,80 | - |
| Bernsteinsäure | - | 1,10 |
| WWS | 0,45 | 0,55 |
| Parfum | q.s. | q.s. |
| Wasser, NaOH 10-%ig ad pH 5 | -----ad 100,00------ | |

Beispiel 8 - 9

| Wachsstift (Wasserfrei) | 8 | 9 |
|---|---|---|
| Trilaurin | 38,00 | 38,00 |
| Caprylic/capric Triglyceride ("Miglyol 812®") | 29,50 | 29,50 |
| Glycerylstearat, selbstemulgierend | 8,50 | 8,50 |
| Bienenwachs | 21,00 | 21,00 |
| Adipinsäure | 0,50 | - |
| Azelainsäure | - | 0,60 |
| WWS | 0,80 | 0,60 |
| Parfum | q.s. | q.s. |
| Wasser, NaOH 10-%ig ad pH 5 | -----ad 100,00------ | |

**Patentansprüche**

**1.** Verwendung von Wirkstoffkombinationen aus

I) $\alpha,\omega$-Alkandicarbonsäuren und

II) einem natürlichen Wollwachssäuregemisch oder einem durch Destillation eines natürlichen Wollwachssäuregemisches erhältlichen Wollwachssäuregemisch

als Wirkprinzip gegen Superinfektionen.

**2.** Verwendung nach Anspruch 1, dadurch gekennzeichnet, daß das Wollwachssäuregemisch erhältlich ist aus Rohwollwachssäure durch Kurzwegdestillation im Vakuum bei $10^{-1}$ bar aus dem Destillationstemperaturintervall von 150 - 200° C.

**3.** Verwendung nach Anspruch 1, dadurch gekennzeichnet, daß das durch Destillation erhältliche Wollwachssäuregemisch sich durch folgende Parameter auszeichnet:

| | |
|---|---|
| Tropfpunkt : | 50 - 54° C |
| Säurezahl : | 166 - 170 |
| Verseifungszahl : | 175 - 190 |
| OH - Zahl : | 60 - 80 |
| Jodzahl : | 7 - 12 |

**4.** Verwendung nach Anspruch 1, dadurch gekennzeichnet, daß die α,ω-Alkandicarbonsäure oder -säuren gewählt werden aus der Gruppe der Substanzen, die von der generischen Formel

HOOC-(CH2)n-COOH beschrieben werden,

wobei n Zahlen von 1 bis 8 annehmen kann.

**5.** Verwendung nach Anspruch 1, dadurch gekennzeichnet, daß der Gehalt an

I) α,ω-Alkandicarbonsäuren und

II) einem natürlichen Wollwachssäuregemisch oder einem durch Destillation eines natürlichen Wollwachssäuregemisches erhältlichen Wollwachssäuregemisch

so gewählt wird, daß Verhältnisse von I) und II) wie 5 : 1 bis 1 : 5, insbesondere wie etwa 1 : 1 entstehen.

**6.** Verwendung nach Anspruch 1, dadurch gekennzeichnet, daβ das natürliche oder durch Destillation aufbereitete Gemisch aus Wollwachssäuren in Konzentrationen von 0,05 - 10,00 Gew.-%, bevorzugt 0,1 - 5, 0 Gew.-%, vorliegt, jeweils bezogen auf das Gesamtgewicht der Zubereitungen.

**7.** Verwendung nach Anspruch 1, dadurch gekennzeichnet, daß die α,ω-Alkandicarbonsäure oder die α,ω-Alkandicarbonsäuren in Konzentrationen von 0,01 - 10,00 Gew.-%, bevorzugt 0,05 - 5,0 Gew.-%, vorliegen, jeweils bezogen auf das Gesamtgewicht der Zubereitungen.

**8.** Verwendung von Wirkstoffkombinationen aus

I) α,ω-Alkandicarbonsäuren und

II) einem natürlichen Wollwachssäuregemisch oder einem durch Destillation eines natürlichen Wollwachssäuregemisches erhältlichen Wollwachssäuregemisch

zur Herstellung einer kosmetischen oder dermatologischen Zubereitung gegen Superinfektionen.